(19) Europäisches Patentamt / European Patent Office / Office européen des brevets

(11) **EP 2 612 960 B1**

(12) **EUROPEAN PATENT SPECIFICATION**

(45) Date of publication and mention
of the grant of the patent:
**31.12.2014 Bulletin 2015/01**

(21) Application number: **11821390.9**

(22) Date of filing: **06.06.2011**

(51) Int Cl.:
*A61F 13/513* (2006.01)  *A61F 13/511* (2006.01)
*D04H 1/22* (2006.01)  *A61F 13/15* (2006.01)
*D04H 1/482* (2012.01)  *D04H 1/485* (2012.01)
*D04H 1/49* (2012.01)  *D04H 1/498* (2012.01)
*A61F 13/537* (2006.01)

(86) International application number:
**PCT/JP2011/063431**

(87) International publication number:
**WO 2012/029372 (08.03.2012 Gazette 2012/10)**

(54) **NONWOVEN FABRIC, METHOD OF MANUFACTURING SAME, AND ABSORBENT ARTICLE**

VLIESSTOFF, VERFAHREN ZU SEINER HERSTELLUNG UND SAUGFÄHIGER ARTIKEL DARAUS

TISSU NON TISSÉ, SON PROCÉDÉ DE FABRICATION ET ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.08.2010 JP 2010194621**

(43) Date of publication of application:
**10.07.2013 Bulletin 2013/28**

(73) Proprietor: **Unicharm Corporation
Ehime 799-0111 (JP)**

(72) Inventors:
• **KIMURA, Akihiro
Kanonji-shi
Kagawa 769-1602 (JP)**

• **OBA, Toru
Kanonji-shi
Kagawa 769-1602 (JP)**

(74) Representative: **Eke, Philippa Dianne et al
Saunders & Dolleymore LLP
9 Rickmansworth Road
Watford Hertfordshire WD18 0JU (GB)**

(56) References cited:
EP-A1- 2 161 361  EP-A2- 0 958 802
WO-A1-2008/156075  JP-A- 11 318 983
JP-A- 2009 000 173  JP-A- 2009 030 218
US-A- 3 653 382

**Description**

<u>Technical Field</u>

The present invention relates to a nonwoven fabric and a method for producing it, and to an absorbent article using the nonwoven fabric, and more specifically it relates to a disposable body fluid-absorbing article such as a disposable diaper or sanitary napkin, and a nonwoven fabric suitable for use as a liquid-permeable sheet in such a body fluid-absorbing article, as well as a production method for the nonwoven fabric.

<u>Background Art</u>

Nonwoven fabrics are used in absorbent articles such as disposable diapers and sanitary napkins, and in particular they are used as top sheets in contact with skin, or as second sheets provided between top sheets and absorbent bodies. One type of nonwoven fabric known in the prior art, as a nonwoven fabric used in such absorbent articles, has heights and recesses alternately arranged to form repeating corrugations in the transverse direction (see PTL 1, for example). The cross-sectional shapes of the heights of the nonwoven fabric described in PTL 1 are roughly semicircular. Since the contact area between the nonwoven fabric and the skin of the wearer is reduced by the heights having such cross-sectional shapes, it prevents redepositing of body fluid over the skin of the wearer when body fluid that has been absorbed into the absorbent body has caused re-wetting under external pressure. Also, the fiber density at the base sections of the recesses is lower than the fiber density at the heights, and this allows body fluid pooling in the recesses to pass through the nonwoven fabric and be rapidly absorbed into the absorbent body.

<u>Citation List</u>

<u>Patent literature</u>

PTL 1 Japanese Unexamined Patent Publication No. 2008-25082

SUMMARY OF THE INVENTION

<u>Technical Problem</u>

With the nonwoven fabric described in PTL 1, since the fiber density is low at the base sections of the recesses, body fluid that has been absorbed into the absorbent body often produces re-wetting through the bases of the recesses under external pressure, pooling in the recesses. When this occurs, and the heights collapse under load applied by the wearer, the body fluid overflows through the recesses and becomes redeposited over the skin of the wearer.

<u>Solution to the Problem</u>

In order to solve the aforementioned problems, the invention employs the following construction. Specifically, the nonwoven fabric of the invention is a nonwoven fabric having a mutually perpendicular longitudinal direction, transverse direction and thickness direction, and having in the thickness direction a front side, and a back side on the side opposite it, having on the front side heights and recesses formed so as to extend in an alternating fashion parallel to each other in the longitudinal direction, to form repeating corrugations in the transverse direction, and on the back side a back side section formed across the entire side, the heights being composed of height tips located at the tips of the heights and neck sections connecting the height tips with the back side section, the cross-sectional widths of the neck sections being narrower than the height tips in a cross-section of the heights in the transverse direction, and having recess spaces formed by the two neck sections supporting each of the adjacent height tips, and the back side section. In a cross-section in the transverse direction of the heights, approximate Ω-shape are formed wherein the cross-sectional widths of the neck sections are narrower than the height tips, and the height tips are preferably the roughly circular sections of the approximate Ω-shapes. Also, preferably when the height tips collapse across the transverse direction by an arbitrary load applied in the thickness direction, adjacent height tips come into close contact with each other so that the recess spaces can be blocked by the height tips, neck sections and back side section. In addition, most of the fibers on the back side section side of the neck sections may be oriented in the thickness direction. Also, the degree of fiber aggregation in the neck sections is preferably higher than the degree of fiber aggregation in the height tips. Furthermore, the height tips may have a first fiber layer composed of first fibers, the back side section may have a second fiber layer composed of second fibers, that develop crimping by heat treatment at a temperature at which the first fibers do not develop crimping and fusion at the intersections of the first fibers does not occur, and the neck section may have a first fiber layer except on a portion of the back side section side, and a second fiber layer on the back side section side. Another mode of the invention is an absorbent article wherein any of the aforementioned nonwoven fabrics is used as a top sheet which is to contact with the skin of a wearer, or a second sheet to be situated between a top sheet and an absorbent body. Yet another mode of the invention is a method for producing a nonwoven fabric, comprising a web-forming step in which a second web is formed composed of second fibers that develop crimping by heat treatment, a web-layering step in which a first web is formed composed of first fibers that do not develop crimping at the temperature at which the second fibers develop crimping and that do not undergo fusion at the fiber intersections, and the first web is layered on the second web, a recess-forming step in which the first fibers composing the first web and the second fibers composing the second web are divided in the CD direction by a fluid stream, and a plurality of recesses are formed extending parallel to a prescribed direction to a depth reaching from the first web into the second web, a heating step in which crimping is produced in the second fibers of the second web by heating, and a fusing step in which the intersections between the first fibers are fused, wherein in the recess-forming step there are created sections where the degree of fiber aggregation of the second fibers is increased directly under the heights formed by the recesses, and in the heating step there are created neck sections in the heights by developing latent crimping by heating at the sections that have an increased degree of fiber aggregation of the second fibers directly under the heights formed by the recesses. In the heating step, preferably the stack of the first web and second web layered in the web-layering step is stretched to a prescribed stretch factor, the shrinking effect of the second fibers taking place selectively in the CD direction so that the second fibers become bundled directly under the heights. Also, in the recess-forming step, the blowing direction of the nozzle of the blower that produces the fluid stream may be pointed in the direction opposite the traveling direction of the stack of the first web and second web, to orient the constituent fibers of the stack in the CD direction.

Advantageous Effect of the Invention

According to the invention it is possible to minimize "rewet-back" in which fluid absorbed into the absorbent body exudes out to the front side, even when a load is applied to the nonwoven fabric by the wearer.

Brief Description of the Drawings

Fig. 1(a) is a perspective view schematically showing a portion of a nonwoven fabric according to an embodiment of the invention, and Fig. 1(b) is a cross-sectional view of the nonwoven fabric along A-A' of Fig. 1(a).
Fig. 2 is a drawing showing an example of a step for producing a nonwoven fabric according to an embodiment of the invention.

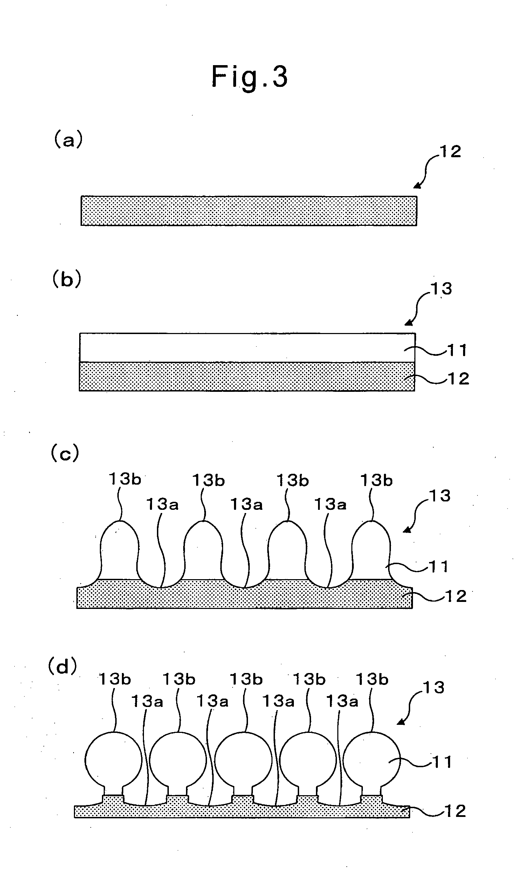

Fig. 3(a) is a drawing showing a portion of a cross-section of the second web cut in the transverse direction (CD direction), as seen from the longitudinal direction (MD direction), Fig. 3(b) is a drawing showing a portion of a cross-section of the stack of the first web and second web cut in the CD direction, as seen from the MD direction, Fig. 3(c) is a drawing showing a portion of a cross-section of the recess-formed stack of the first web and second web cut in the CD direction, as seen from the MD direction, and Fig. 3(d) is a drawing showing a portion of a cross-section of the heat-treated stack cut in the CD direction, as seen from the MD direction.
Fig. 4 is a drawing illustrating a step of blowing a heated fluid stream from a nozzle of a blower onto a stack.
Fig. 5 is a drawing illustrating nozzles of a blower.
Fig. 6 is a drawing illustrating the blowing direction for a nozzle of a blower.
Fig. 7 is a drawing illustrating the principle by which a nonwoven fabric according to an embodiment of the invention minimizes rewet-back.
Fig. 8 is a photograph illustrating the structure of a nonwoven fabric A.
Fig. 9 is a photograph illustrating a sample in a rewet-back test.
Fig. 10 is a diagram showing measurement results for the dimensions of the constituent sections of a nonwoven fabric A.
Fig. 11 is a photograph showing the results of a recess load test for a nonwoven fabric A.
Fig. 12 is a perspective view showing an acrylic board used for measurement of air permeation volume in the horizontal direction.
Fig. 13 is a general schematic view showing a Gurley tester used for measurement of air permeation volume in the horizontal direction.

Description of Embodiments

Embodiments of the invention will now be explained in detail with reference to the accompanying drawings.

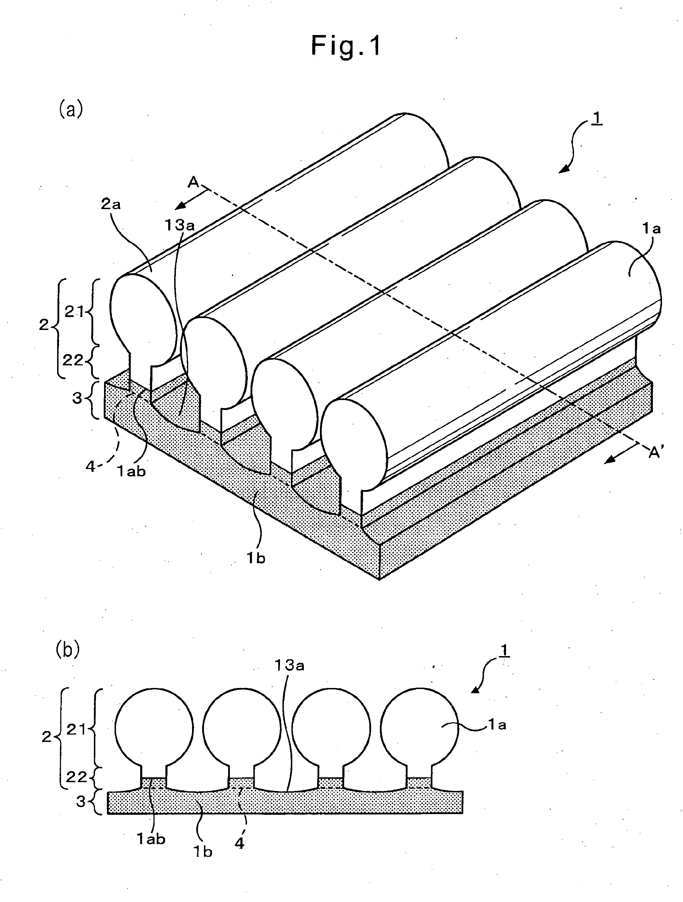

Fig. 1 is a pair of perspective views schematically showing a portion of a nonwoven fabric 1 according to an embodiment of the invention. The nonwoven fabric 1 is composed of a plurality of heights 2 arranged parallel at approximately equal spacings, and a back side section 3 that forms the foundation of the plurality of heights 2. The heights 2 extend in the machine direction, i.e., the longitudinal direction (MD direction) of the nonwoven fabric 1. The longitudinal direction will hereunder be referred to as the "MD direction". The direction perpendicular to the MD direction will hereunder be referred to as the cross-machine direction, or the transverse direction (CD direction). The spacings between the heights 2 do not necessarily need to be roughly equal spacings, and they may differ. Of the two opposing main surfaces of the nonwoven fabric 1, the surface on which the heights 2 are formed is the front side of the nonwoven fabric 1, and the opposite side is the back side of the nonwoven fabric 1. The front side of the nonwoven fabric 1 has heights 2 and recesses 13a formed in an alternating orientation, extending parallel to each other in the MD direction, to form repeating corrugations in the CD direction. A back side section 3 is formed across the entire side on the back side of the nonwoven fabric 1.

The cross-sectional shape of the heights 2 is an approximate $\Omega$-shape, as cut in the CD direction. An "approximate $\Omega$-shape" is a shape constituted by a combination of a roughly circular shape and a roughly rectangular shape. The roughly circular sections on the tip ends of the heights 2 will hereunder be referred to as "height tips 21", and the narrow-width rectangular-shaped sections on the back side section 3 side will hereunder be referred to as "neck sections 22". The term "roughly circular shape" includes not only circular shapes but also ellipsoid shapes. It also includes biased circular and ellipsoid shapes. The term "roughly rectangular shape" refers to a rectangular or square shape, or their biased forms, and it includes those whose sides are curved, such as convex or concave sides.

When pressure is not being applied to the heights 2 in the thickness direction of the nonwoven fabric 1, the height tips 21 of each of the two adjacent heights 2 may contact each other, but preferably they are not in contact. Also, when pressure is being applied to the heights 2 in the thickness direction of the nonwoven fabric 1, the height tips 21 undergo deformation preferentially over the neck sections 22, becoming flattened, and preferably the height tips 21 of the adjacent heights 2 come into contact or overlap and support each other. Also, it is preferred that the neck sections 22 undergo minimal deformation even when pressure is applied to the heights 2 in the thickness direction. This can ensure spaces between the recesses 13a formed by the two adjacent neck sections 22 and the back side section 3.

The distance between the tips 2a of each two adjacent heights 2 is preferably 1.00 to 8.00 mm and more preferably 2.00 to 7.00 mm. The "tips 2a" are the apex sections of the heights 2, in a cross-section of the heights 2 in the CD direction. If the distance between the tips 2a of each two adjacent heights 2 is smaller than 1.00 mm it may be difficult to form approximate $\Omega$2-shapes in the cross-section of the heights 2, while if it is larger than 8.00 mm, the adjacent height tips 21 may not contact each other even when the height tips 21 of the heights 2 collapse across the CD direction due to load by the wearer, and it may become impossible to block the spaces between the recesses 13a by the height tips 21, the neck sections 22 and the back side section 3.

In the recesses 13a formed by each two adjacent heights 2 and the back side section 3, the distance between a line connecting the tips 2a of the two adjacent heights 2 and the base of the recesses 13a, i.e. the depth of the recesses 13a, is preferably 0.40 to 9.00 mm and more preferably 0.70 to 4.50 mm. If the depth of the recesses 13a is smaller than 0.40 mm, body fluid may not be well retained in the recesses 13a, and if it is larger than 9.00 mm the heights 2 will tend to become inclined under load of the wearer, which can potentially cause collapse of the recesses 13a.

The cross-sectional width of the height tips 21 of the heights 2, in a cross-section of the heights 2 in the CD direction, is not particularly restricted so long as it is no greater than the distance between the tips 2a of each two adjacent heights 2, but it is preferably 1.00 to 5.00 mm. If the cross-sectional width of the height tips 21 is larger than the distance between the tips 2a of each two adjacent heights 2, the height tips 21 will be covering the adjacent heights 2, and this may interfere with retention of body fluid from the wearer in the recesses 13a formed by the two adjacent heights 2 and the back side section 3. If the cross-sectional width of the height tips 21 is smaller than 1.00 mm, the adjacent height tips 21 may not contact each other even when the height tips 21 collapse under load by the wearer, and it may become impossible to block the spaces between the recesses 13a by the height tips 21, the neck sections 22 and the back side section 3. Also, if the cross-sectional width of the height tips 21 is larger than 5.00 mm, the height tips 21 of the heights 2 will undergo greater collapse, and the recesses 13a formed by the two adjacent heights 2 and the back side section 3 may potentially become filled by the height tips 21.

The cross-sectional width of the neck sections 22 of the heights 2 is preferably 0.30 to 4.80 mm, although this is not particularly restrictive so long as the cross-sectional width of the neck sections 22 is narrower than the height tips 21 of the heights 2 in a cross-section of the heights 2 in the CD direction. If the cross-sectional width of the neck sections 22 is larger than the cross-sectional width of the height tips 21, an approximate $\Omega$-shape will not be formed in the heights 2. If the cross-sectional width of the neck sections 22 is smaller than 0.30 mm, the neck sections 22 will become inclined under load by the wearer, often causing collapse of the recesses 13a formed by the two adjacent heights 2 and the back side section 3.

Also, the ratio of the cross-sectional width of the height tips 21 of the heights 2 with respect to the cross-sectional width of the neck sections 22 of the heights 2, in a cross-section of the heights 2 in the CD direction, is preferably between 1.04 and 16.60. If this ratio is smaller than 1.04, it may become difficult to ensure spaces between the recesses 13a formed by the two adjacent heights 2 and the back side section 3, even when the height tips 21 have collapsed and the adjacent height tips 21 are supporting each other. If the ratio is greater than 16.60, the spaces between the recesses 13a formed by the two adjacent heights 2 and the back side section 3 will become too small depending on the height tips 21 of the collapsed heights 2, such that body fluid of the wearer may not become significantly retained.

Also, the distance between the tips of the heights 2 and the back side of the nonwoven fabric 1 (the side opposite the height 2 side of the back side section 3), i.e. the thickness of the nonwoven fabric 1, is preferably 0.50 to 10.00 mm and more preferably 0.80 to 5.00 mm. If the thickness of the nonwoven fabric 1 is smaller than 0.50 mm, the spaces between the recesses 13a formed by the two adjacent heights 2 and the back side section 3 will become too small, such that body fluid of the wearer may not become significantly retained. If the thickness of the nonwoven fabric 1 is larger than 10.00 mm, the nonwoven fabric 1 will become a little stiffer overall, and the nonwoven fabric 1 may fail to fit the skin of the wearer.

The degree of fiber aggregation of the fibers in the neck sections 22 of the heights 2 is preferably higher than the degree of fiber aggregation of the fibers in the height tips 21. This will cause the height tips 21 to collapse preferentially before the neck sections 22, under load of the wearer. The "degree of fiber aggregation" is the same as the proportion of fibers per unit volume. The orientation of the fibers in the neck sections 22 is preferably in the thickness direction of the back side section 3. A high degree of fiber aggregation of the fibers in the neck sections 22, and/or a fiber orientation in the thickness direction of the back side section 3, on the back side section 3 side of the neck sections 22, will help the heights 2 to be resistant to inclination under load by the wearer, and can prevent collapse of the neck sections 22.

In the heights 2, the height tips 21 and the sections other than a portion of the back side section 3 side of the neck sections 22 are composed of the first fiber layer 1a, while a portion of the back side section 3 side of the neck sections 22 and the back side section 3 are composed of the second fiber layer 1b. Since the second fiber layer 1b has a higher degree of fiber aggregation and is more rigid than the first fiber layer 1a, as explained below, the heights 2 are resistant to being inclined under load by the wearer, and the recesses 13a formed by each two adjacent neck sections 22 and the back side section 3 are resistant to collapse.

The distance from the border 1ab between the first fiber layer 1a and the second fiber layer 1b to the border 4 between the heights 2 and the back side section 3 is preferably no greater than 25.00% and more preferably 5.00 to 20.00% of the distance from the tips 2a of the heights 2 to the border 4 between the heights 2 and the back side section 3. If this is greater than 25.00%, the nonwoven fabric 1 will become a little stiffer overall and will not easily fit the skin of the wearer, while the cushioning properties of the nonwoven fabric 1 may also be reduced.

Depending on the first fiber layer 1a, the heights 2 may be composed entirely of the first fiber layer 1a, and the back side section 3 may be composed entirely of the second fiber layer 1b. Also, depending on the first fiber layer 1a, a portion of the heights 2 and the height 2 side of the back side section 3 may be composed of the first fiber layer 1a, while the rest of the back side section 3 is composed of the second fiber layer 1b. Alternatively, the height tips 21 may be composed entirely of the first fiber layer 1a and the neck sections 22 may be composed entirely of the second fiber layer 1b.

The first fiber layer 1a comprises fibers whose intersections are heat-fused. Examples of such fibers include fibers composed of thermoplastic polymer materials, including polyolefins such as polyethylene and polypropylene, polyesters such as polyethylene terephthalate, and polyamides. Core-sheath or side-by-side composite fibers, composed of combinations of multiple thermoplastic polymer materials, may also be used for such fibers.

The first fiber layer 1a preferably develops essentially no latent crimping by heat in the heat treatment step when the nonwoven fabric 1 is formed, or it develops essentially no latent crimping by heating at the temperature at which the second fiber layer 1b develops latent crimping by heating, as described below. Even if it undergoes heat shrinkage, the first fiber layer 1a preferably has low heat shrinkage compared to the second fiber layer described below.

The second fiber layer 1b is a fiber layer that develops three-dimensional crimping of the constituent fibers in a coil shape by heat treatment. By using fibers with such a latent crimping property, it is possible, by heat treatment, to easily shrink the web composed of the second fiber layer 1b and easily increase the degree of fiber aggregation in the second fiber layer 1b. Examples of fibers that can suitably develop three-dimensional crimping in the second fiber layer 1b include eccentric core-sheath composite fibers and side-by-side composite fibers, whose components are two or more different thermoplastic resins with different shrinkage factors.

The fiber size (fineness) of the fibers of the first fiber layer 1a and second fiber layer 1b is preferably 0.7 to 20.0 dtex and more preferably 2.2 to 6.6 dtex. A fiber size of smaller than 0.7 dtex will hamper web formation by carding, while a fiber size of larger than 20.0 dtex will result in fewer fibers per unit volume, in which case web formation may become difficult.

The fiber length of the fibers of the first fiber layer 1a and second fiber layer 1b is preferably 20 to 80 mm, in order to facilitate random orientation of the fibers and direct web shrinkage as much as possible in the CD direction. A fiber length of shorter than 20 mm may result in poor carding properties and may hamper web formation, while a fiber length of longer than 80 mm will increase the tangling force between fibers in the web state, thus potentially lowering the fiber re-orientation efficiency in the step of forming the recesses described below (step C) and hampering formation of the heights 2.

The basic weight of the fibers of the first fiber layer 1a and second fiber layer 1b is preferably 5 to 50 g/m$^2$ and more preferably 10 to 30 g/m$^2$. A basic fiber weight of lower than 5 g/m$^2$ may promote re-wetting of body fluid that has been absorbed into the absorbent body, when it is subjected to external pressure, while a basic fiber weight of greater than 50 g/m$^2$ may impair the liquid-permeability of the first fiber layer 1a and second fiber layer 1b. The fiber basis weights of the first fiber layer 1a and the fiber basis weight of the second fiber layer 1b may be either the same or different.

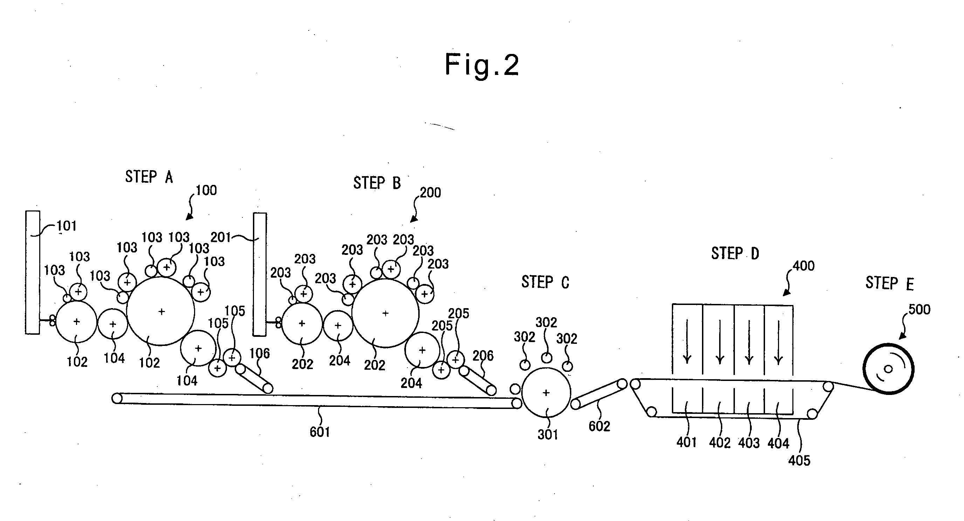

A suitable production method for a nonwoven fabric 1 according to an embodiment of the invention will now be described with reference to Fig. 2. This production method is merely an example of the production method of the invention, and is not meant to be limitative.

Fig. 2 is a drawing showing an example of a step for producing a nonwoven fabric 1 according to an embodiment of the invention. The step of producing the nonwoven fabric 1 is composed of steps A to E.

Step A

In step A, a roller card 100 is used to prepare a second web for formation of the second fiber layer 1b. The staple fibers used to form the second fiber layer 1b are passed through preparation steps such as a bale breaking, fiber splitting and combination, and are then sent to a feeder 101 and supplied from the feeder 101 to the roller card 100. At the feeder 101, the supply of staple fibers is regulated so as to maintain a constant weight and/or volume of staple fibers supplied to the roller card 101.

The staple fibers supplied to the roller card 100 are opened at the roller card 100 and randomly oriented, to form the second web. The roller card 100 has a cylinder 102, a walker 103, a doffer 104 and a condenser 105, and opening of the staple fibers is accomplished mainly between the cylinder 102 and the walker 103, while the opened and sheeted staple fibers, i.e., the web, is sent to the doffer 104. The web is then further randomized in its orientation by delivery of the staple fibers at the doffer 104 and condenser 105, and then sent to a conveyor 106 and transported on a conveyor 601. The second web 12 situated on the conveyor 601 is illustrated in Fig. 3(a). Fig. 3(a) shows a portion of a cross-section of the web 12 cut in the CD direction, as viewed from the MD direction.

Step B

In step B, a roller card 200 is used to prepare a first web for formation of the first fiber layer 1a. Preparation of the first web in step B is carried out by the same method as preparation of the second web in step A, and therefore preparation of this web will not be explained again. The first web prepared in step B is layered on the second web 12 situated on the conveyor 601, and it is transported together with the second web 12 to step C. The first web 11 layered on the second web 12 will hereunder be referred to as the "stack". The stack 13 is illustrated in Fig. 3(b). Fig. 3(b) shows a portion of a cross-section of the stack 13 cut in the CD direction, as viewed from the MD direction.

Step C

In step C, recesses are formed on the front side on the first web 11 side of the stack 13, extending in the MD direction and arranged parallel to the CD direction at approximately equal spaces. In some cases, the recess spacings will not need to be approximately equal spaces. The plurality of recesses may have consistent depths, or their depths may differ.

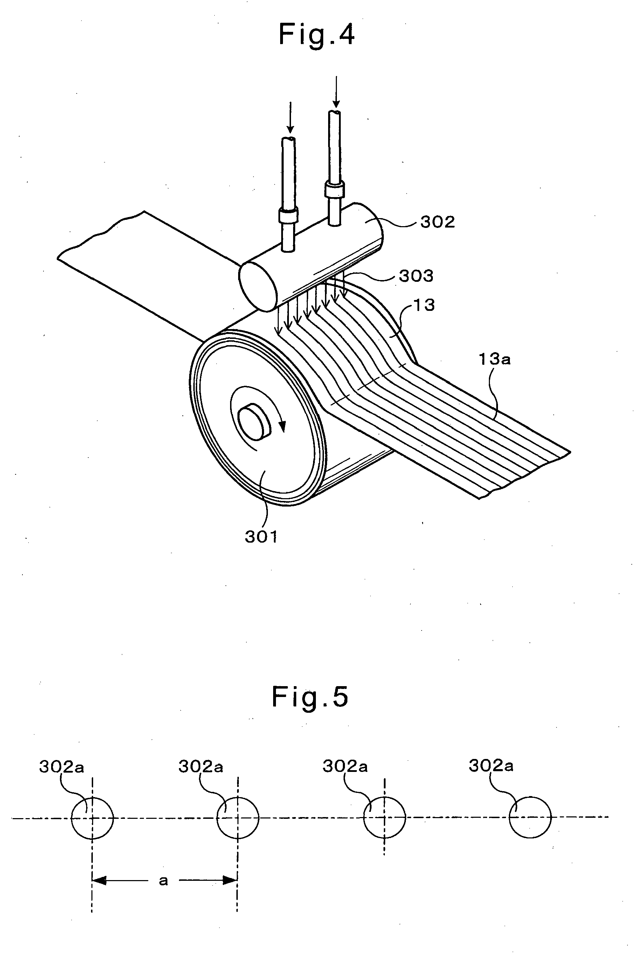

As shown in Fig. 2, the stack 13 comprising the first web 11 and the second web 12 is then transported from the conveyor 601 to a suction drum 301. Also, as shown in Fig. 4, a heated fluid stream 303 is blown from the nozzle of a blower 302 to divide the fibers 11 of the first web and a portion of the second web 12 of the stack 13, to form recesses 13a. The state of the recesses 13a formed by the fluid stream 303 will now be explained with reference to Fig. 3(c). Fig. 3(c) shows a portion of a cross-section of the recess 13a-formed stack 13 cut in the CD direction, as viewed from the MD direction. The fibers divided in the CD direction by the fluid stream 303 collect at the sides of the base ends of the recesses 13a, i.e., directly under the heights 13b, and therefore the degree of fiber aggregation increases near the sides of the base ends of the recesses 13a, i.e., directly under the heights 13b. As mentioned above, a portion of the second web 12 is also divided, and therefore the constituent fibers of the divided second web 12 also collect on the sides of the base ends of the recesses 13a, i.e., directly under the heights 13b. As a result, contraction of the heights 13b on the root ends is increased in the subsequent heat treatment. Consequently, neck sections 22 are easily formed at the middle sections in the thickness direction of the height tips and the second fiber layer. Three blowers 302 are used as shown in Fig. 2, but only one blower 302 is shown in Fig. 4 for simplicity of illustration.

The recesses 13a of the stack 13 reach to the interior of the second web 12.

The main purpose in step C is movement of the fibers by spraying with the fluid stream 303, and formation of

# EP 2 612 960 B1

a height/recess structure by the fiber movement, and preferably tangling and fusion of the fibers by spraying with the fluid stream 303 is kept to a minimum in order to avoid inhibiting the contractive force of the second web during the heat treatment described below.

The temperature of the fluid stream 303 blown from the nozzle of the blower 302 is preferably 100°C to 350°C. If the temperature of the fluid stream is lower than 100°C the fibers will not be significantly softened, and therefore movement of the fibers may be lessened and it may not be possible to modify the molded shape by heat, while if the temperature is higher than 350°C, tangling and fusion of the fibers may progress under heating, and the contractive force of the second web may be inhibited during the heat treatment described below.

As shown in Fig. 5, the nozzle hole pitch (denoted as "a") for the nozzles 302a of the blower 302 is preferably at least 2.0 mm and no greater than 10.0 mm. If the nozzle hole pitch is smaller than 2.0 mm, the number of recesses 13a formed in the stack 13 per unit length in the CD direction will be increased, resulting in a smaller amount of fibers gathering at the neck section 22 of each height 2, and thus failure to form approximate Ω-shapes by heights 2 after the heat treatment described below. Also, if the nozzle hole pitch is larger than 10.0 mm the width of the heights 2 in the CD direction may become too large.

The flow rate of the fluid stream 303 blown from the nozzles 302a of the blower 302 is preferably 8 to 11 liter/min for each nozzle hole. If the flow rate of the fluid stream 303 is less than 8 liter/min per nozzle hole it may not be possible to form recesses 13a of sufficient depth in the stack 13, while if the flow rate of the fluid stream 303 is greater than 11 liter/min per nozzle hole, the texture of the web (stack 13) may be vitiated by the fluid stream.

The fluid stream 303 blown from the nozzles 302a of the blower 302 may be a gas such as air or water vapor, or it may be a liquid such as water. It may also be an aerosol of a solid or liquid fine particles in a gas.

Step D

In step D, first heat treatment is conducted at a temperature at which the constituent fibers of the first web 11 do not develop crimping and the fiber intersections do not fuse, the heat treatment causing development of latent crimping of the constituent fibers of the second web 12 and resulting in heat shrinkage of the second web 12, and this is followed by heat treatment at an even higher temperature to cause fusion of the second web 12 and the first web 11 or the fiber intersections of the constituent fibers of the first web 11. The heat treatment of the stack 13 is carried out in a heat setting machine 400, as shown in Fig. 2. The heat setting machine 400 has four heat setting chambers 401-404, and the stack 13 is moved into each of the heat setting chambers 401-404 by a conveyor 405. In each setting chamber 401-404, hot air at a prescribed temperature is blown toward the stack 13.

The stack 13 with the recesses 13a formed in step C is transported into the heat setting machine 400 by a conveyor 602. The stack 13 is then transported into the heat setting chambers 401,402 by the conveyor 405. At the heat setting chambers 401,402, the stack 13 is heated to develop crimping of the latent crimped fibers in the constituent fibers of the second web (heat setting machine introduction step). The temperature for the heat treatment is below the melting point of the heat-fusible components of the first web 11, and a temperature at which the constituent fibers of the second web develop latent crimping. For example, the temperature for heat treatment in the heat setting chambers 401,402 is 90°C to 130°C.

The web shrinkage factor for the fibers that develop latent crimping by heating in the second web 12 is measured by the following method, and it is preferably at least 40%.

Area shrinkage factor of web

(1) A 200 g/m$^2$ web is produced with 100% of the fibers to be measured.
(2) It is cut to the prescribed length and width, and the area is measured. In order to reduce measurement error, it is preferably cut to about 250 mm x 250 mm. The measured area before shrinkage is designated as "a".
(3) The cut web is allowed to stand for 5 minutes in an oven adjusted to 145°C.
(4) The length and width of the heat-treated web after shrinkage are measured and the area is calculated. The measured area after shrinkage is designated as "b".
(5) The area shrinkage factor is calculated by the following formula based on the areas before and after heat shrinkage.

$$\text{Area shrinkage factor (\%)} = (a-b)/a \times 100$$

Latent crimping of the second web that develops by heat treatment causes shrinkage of the stack 13 in the CD

7

direction. On the other hand, the first web 11 undergoes little or absolutely no shrinkage, but contractive force of the second web 12 causes deformation near the border between the first web 11 and the second web 12 to produce shrinkage in the CD direction. As mentioned above, the constituent fibers of the divided second web 12 collect on the sides of the base ends of the recesses 13a, and therefore shrinkage occurs significantly near the bases of the recesses 13a, while application of a prescribed tensile force to the sheet during the heating step causes the fibers to bundle directly under the heights and selectively produces a shrinking effect in the CD direction, such that the cross-sectional shapes of the heights formed by the recesses 13a are converted into approximate $\Omega$-shape. Also, the degree of fiber aggregation of the second web 12 is much higher than the first web 11, due to the heat shrinkage and tension effect.

The stack 13 that has undergone shrinkage in the CD direction is transported to the heat setting chambers 403,404 by the conveyor 405. At the heat setting chambers 403,404, heat treatment is carried out and fusion occurs at the intersections of the constituent fibers of the first web 11 (heat setting machine exiting step). The heat treatment temperature at this time is not restricted so long as it is a temperature at which the constituent fibers of the first web fuse, and it may be 130°C to 150°C, for example.

The step of fusing the intersections of the constituent fibers of the web is not limited to fusion by heat treatment, and a step of bonding the intersections of the constituent fibers of the web may employ another web bonding technique such as chemical bonding, needle punching, water jet punching or stitch bonding.

A stack 13 heat treated in step D is shown in Fig. 3(d). Fig. 3(d) shows a portion of a cross-section of a heat-treated stack 13 cut in the CD direction, as viewed from the MD direction. In the stack 13 in step D, the cross-sections of the heights formed by the recesses 13a are approximate $\Omega$-shapes. The recesses 13a formed in the stack 13 reach into the second web 12. Since the second web 12 has a higher degree of fiber aggregation and is more rigid, the root sections of the heights formed by the recesses 13a will be more robust if the recesses 13a reach into the web 12. Also, contractive force of the second web 12 causes the orientation of the fibers near the interface between the first web 11 and the second web 12 to change, such that most of the fibers near the interface between the first web 11 and the second web 12 become oriented in the thickness direction of the stack 13.

The heat-treated stack 13 corresponds to the nonwoven fabric 1 shown in Fig. 1, the first web 11 corresponds to the first fiber layer 1a and the second web 12 corresponds to the second fiber layer 1b (see Fig. 1). The heights formed by the recesses 13a correspond to the heights 2, and the sections where the recesses 13a do not reach the second web 12 correspond to the back side section 3.

Step E

In step E, the prepared nonwoven fabric 1 is taken up with a take-up drum 500.

A suitable production method for a nonwoven fabric 1 according to an embodiment of the invention may have further modified production conditions, as follows.

Stretch factor

The stretch factor is the speed of each subsequent step with 100% as the speed of the previous step, and for example, a stretch factor of 10% means a speed of 110% in the subsequent step with respect to the speed in the previous step. In order for the constituent fibers of the second web 12 to bundle directly under the heights 13b and development of crimping of the latent crimped fibers of the second web 12 to be maximized in the CD direction, the following method may be employed. The second web 12 is packed with the doffer 104 and condenser 105 of the roller card 100 (see Fig. 2), and the second web 12 whose fibers are arranged in the MD direction are first randomly arranged. The process advances from step B to step D without significant pulling of the second web 12. In this manner, it is possible for the stack 13 to be moved to the heat treatment step of step D with the latent crimped fibers of the second web 12 kept in a random orientation.

To maintain a random orientation for the constituent fibers of the second web 12, the line conditions may be such that the stretch factor is -5.0 to +5.0% during each of the steps B to D and during transport between steps B to D. If the stretch factor is lower than -5.0%, the stack 13 will become excessively packed in the MD direction and the stack 13 sheet will sag, while if it is greater than +5.0%, the constituent fibers of the second web that develop latent crimping by the heating in step D will become oriented in the MD direction, thus often reducing shrinkage in the CD direction during heat treatment.

During the time between exiting of the stack 13 from the heat setting machine 400 and its take-up onto the take-up drum 500, the stretch factor of the take-up drum 500 with respect to the 602 conveyor may be, for example, 3.0 to 20.0% to selectively orient shrinkage in the CD direction. If the stretch factor is lower than 3.0%, the second web will no longer be able to bundle directly under the heights, and as a result the cross-section of the heights in the CD direction will fail to form a roughly $\Omega$-shape, while if the stretch factor is higher than 20.0%, problems such as tearing of the sheet may occur.

Blower nozzle blowing direction

The constituent fibers of the stack 13 oriented in the MD direction are randomly oriented, and in order to further orient the constituent fibers of the stack 13 more in the CD direction, the blowing direction of the nozzles 302a of the blower 302 may be directed slightly in the direction against where the constituent fibers of the stack 13 are to be oriented in the MD direction, i.e. the direction opposite to the direction in which the stack 13 moves by the suction drum 301 (the direction of the take-up drum 500, or in other words, the traveling direction of the stack 13).

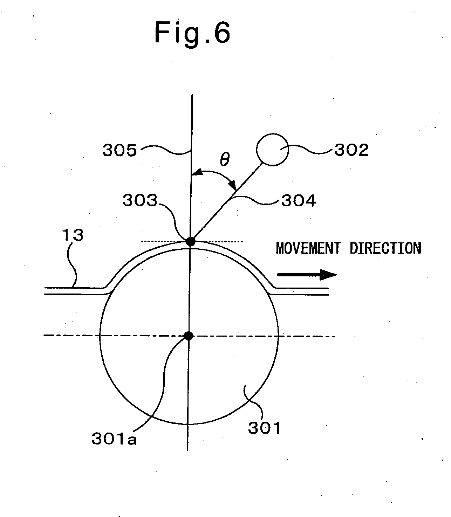

For example, the blowing direction of the nozzles of the blower may be the direction shown in Fig. 6. In Fig. 6, numeral 301a denotes the axial center of the suction drum 301, and numeral 303 denotes the intersection between a line 304 extended in the blowing direction from the nozzle of the blower 302, and the front side of the stack 13. The blowing direction of the nozzles of the blower 302 may be directed in the direction opposite the movement direction of the stack 13, in such a manner that the angle $\theta$ between the line 304 and a line 305 connecting the center 301a of the suction drum 301 and the intersection 303 (hereunder referred to as "nozzle inclination angle") is 1-5°. If the nozzle inclination angle is smaller than 1°, the effect of orienting the constituent fibers of the first web 11 in the CD direction will be minimal, while if the nozzle inclination angle is larger than 5°, the texture of the first web may be vitiated and it may not be possible to form a structure as a nonwoven fabric.

Spraying speed for heated air in heat treatment step

(step D)

In the heat treatment step of step D, the spraying speed for the heated air in the heat setting chambers 401-404 may be 0.1 to 2.0 m/sec, in order to maximize the degree of development of latent crimping by heat treatment of the randomly oriented latent crimped fibers of the second web 12. If the spraying speed for the heated air is lower than 0.1 m/sec, it will not be possible to transfer thermal energy to the second web, and it will therefore be difficult to develop crimping in the fibers by heating of the second web 12, while if the spraying speed for the heated air is higher than 2.0 m/sec, wind pressure will become a factor during development of crimping of the latent crimped fibers of the second web 12, often making it difficult for shrinkage to move toward the CD direction. The spraying speed for the heated air in the heat setting chambers 401-404 may be the same in all of the heat setting chambers 401-404, or it may differ between the heat setting chambers 401-404.

The nonwoven fabric 1 prepared in the manner described above is suitable for use as a liquid-permeable sheet for an absorbent article, and it is especially suitable for use as a top sheet or second sheet in an absorbent article, such as a disposable diaper or sanitary napkin, or a body fluid-absorbing article for incontinence patients.

A "top sheet" is the sheet of the absorbent article that contacts the skin of the wearer, and a "second sheet" is a sheet situated between the top sheet and absorbent body in an absorbent article.

The following is conjectured to be the principle by which the nonwoven fabric 1 minimizes "rewet-back", i.e., action in which fluid absorbed into the absorbent body exudes out to the front side. However, it is to be understood that the invention is not limited to this principle, and minimization of rewet-back by embodiments of the invention may be based on a different principle.

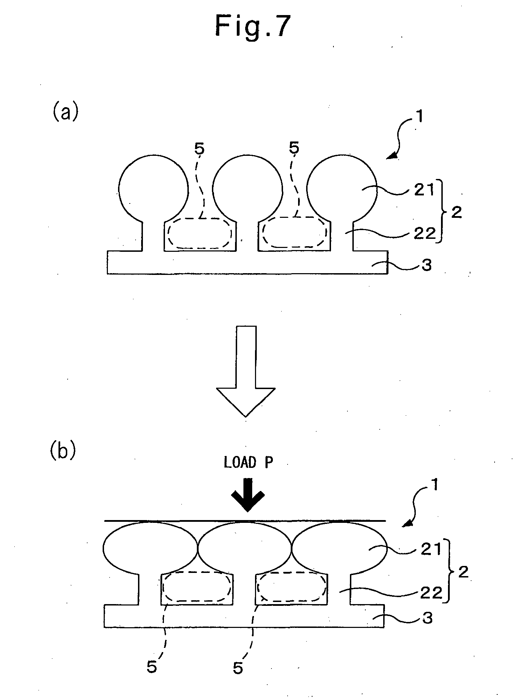

As shown in Fig. 7(a), when no load is applied to the nonwoven fabric 1, the recess spaces 5 formed by each two adjacent heights 2 and the back side section 3 are open on the height tip 21 sides. This allows body fluid from the wearer to pool in the recess spaces 5. However, when a load P is applied to the nonwoven fabric 1, as shown in Fig. 7(b), the height tips 21 collapse across the transverse direction, and the recess spaces formed by each two adjacent neck sections and the back side section are maintained while the adjacent deformed height tips 21 support each other. This causes body fluid absorbed into the absorbent body to be temporarily held in the spaces even when the body fluid re-wets under the load P. Consequently, using a nonwoven fabric 1 as the top sheet and second sheet of an absorbent article blocks re-wetted body fluid from reaching the front side of the top sheet, making it possible to prevent re-wetted body fluid from contacting the skin of the wearer.

Examples

Example 1

Evaluation results for a nonwoven fabric according to an embodiment of the invention will now be presented by examples. These examples are not intended to restrict the scope of the invention.

Preparation of nonwoven fabric

Nonwoven fabric A according to an embodiment of the invention was prepared by the steps illustrated in Fig. 2 for the evaluation described below.

In step A, a second web was prepared with polypropylene/polyolefin-polypropylene copolymer latent crimping side-by-side composite fibers as the constituent fibers. The fiber size of the constituent fibers of the second web was 2.2 dtex, the fiber length was 51 mm, the thickness was 10.0 mm and the basis weight was 20 g/m$^2$ The fibers of the second web had a web area shrinkage factor of 80%.

Next, in step B, a first web was prepared with polyester/polyethylene core-sheath composite fibers as the constituent fibers, and this was layered on the second web. The fiber size of the constituent fibers of the first web was 3.3 dtex, the fiber length was 51 mm, the thickness was 10.0 mm and the basis weight was 20 g/m$^2$. The first web does not develop latent crimping or shrink even when heat-treated at the temperature at which the second web develops latent crimping. The stretch factor from step B to step C was 1.0%, and the stretch factor from step C to step D was 1.0%.

In step C, recesses were formed on the front side on the first web side of the stack comprising the first web and the second web, extending in the MD direction and arranged parallel to the CD direction at approximately equal spacings. The constituent fibers of the stack 13 oriented in the MD direction are randomly oriented, and in order to further orient the constituent fibers of the stack 13 more in the CD direction, the blowing direction of the nozzles 302a of the blower 302 was inclined 3° in the direction against where the constituent fibers of the stack 13 were to be oriented in the MD direction, i.e., the direction opposite to the direction in which the stack 13 moved by the suction drum 301 (the direction of the take-up drum 500). The nozzle hole pitch of the blower nozzles was 3.0 mm. The temperature of the hot air comprising air blown from the blower nozzles was 140°C, and the flow rate of the hot air comprising air blown from the blower nozzles was 10 liter/min per nozzle hole.

In step D, after developing latent crimping in the constituent fibers of the second web by heat treatment at a temperature of 110°C and a heated air spraying speed of 0.7 m/sec, the intersections of the constituent fibers of the first web were fused by heat treatment at a temperature of 140°C and a heated air spraying speed of 1.2 m/sec. Also, in order to promote bundling of the fibers directly under the formed heights and selectively direct the shrinking effect in the CD direction, stretching force was applied to the sheet in the heat treatment step. The stretching force was such for stretching of 10.0% by the take-up drum 500 with respect to the conveyor 602 in Fig. 2. In step E, the prepared nonwoven fabric A was taken up with the take-up drum 500.

Nonwoven fabric structure

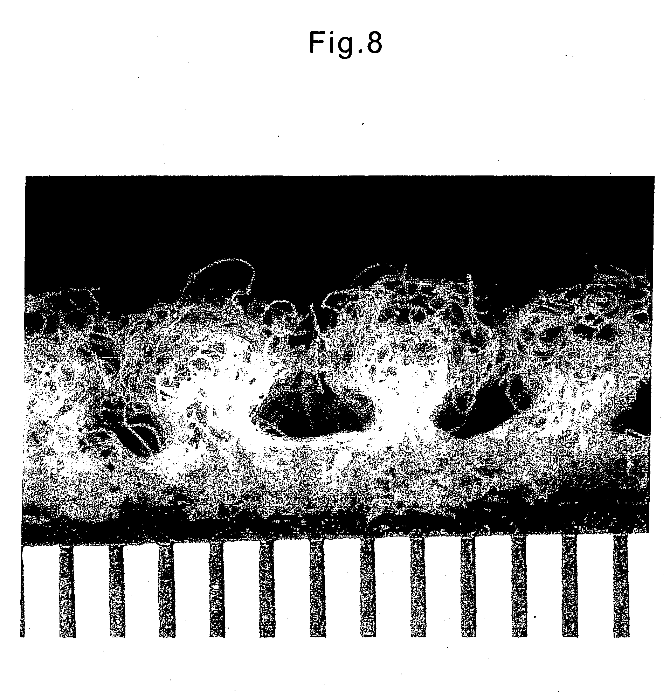

A cross-section of the prepared nonwoven fabric A in the CD direction was photographed at 25x magnification using a VHX-100 digital microscope by Keyence Corp., to examine the structure of the nonwoven fabric A. The results are shown in Fig. 8.

The cross-sectional shapes of the heights in the nonwoven fabric A were confirmed to be approximate Ω-shapes. It was also confirmed that spaces had been formed with inverted approximate Ω-shapes (roughly inverted Ω-shapes) by each two adjacent heights and the back side section. It was further confirmed that the root sections of the heights had the highest degree of fiber aggregation in the heights, and that most of the fibers in the root sections of the heights were oriented in the thickness direction. This was attributed to shrinkage of the second fiber layer directly under the heights occurring in the CD direction. The fiber orientation of the root sections of the heights resulted in the heights forming a rigid structure against compression from above.

Also, the heights had a higher degree of fiber aggregation at the back side section ends than at the tip ends. This resulted in a structure in which the root sections of the heights had a rigid structure resistant to deformation, while the height tips were preferentially deformable under load from the tip ends of the heights. Thus, the recess spaces formed by each two adjacent heights and the back side section were retained even under load.

Upon application of a load to the height tips of the heights, the height tips undergo flattening deformation, and mutual contact or overlapping between the two adjacent height tips that have both undergone flattening deformation results in a mutually supporting structure, such that deformation progresses to a certain extent in the planar direction. Thus, the heights do not collapse any more in the thickness direction beyond that extent. Also, the narrowed sections in which the heights are thinnest in the CD direction are those in which the spaces with roughly inverted Ω-shape are widest in the CD direction.

Rewet-back test

A rewet-back test was conducted for nonwoven fabric A to confirm the effect of minimizing rewet-back of the nonwoven fabric of this embodiment of the invention. The effect of minimizing rewet-back was also examined by a rewet-back test of a comparative example, as a comparison with a conventional nonwoven fabric. The following samples were evaluated.

(1) Nonwoven fabric A

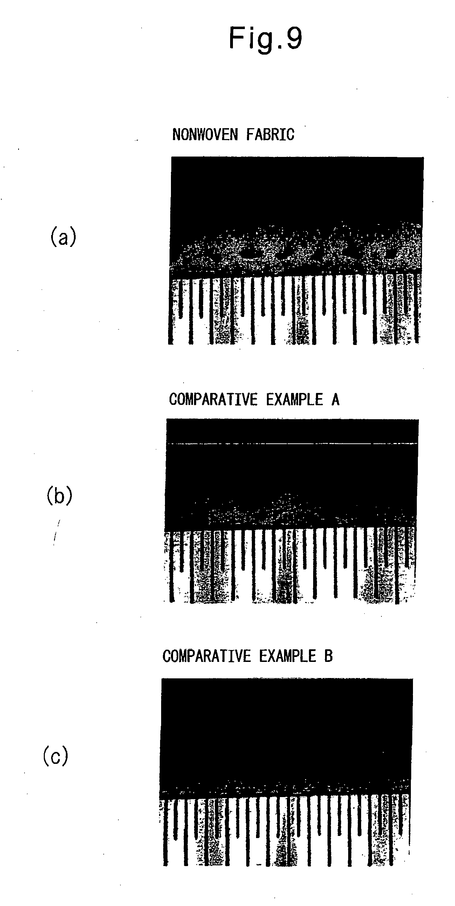

Nonwoven fabric A is a nonwoven fabric according to an embodiment of the invention. A cross-section of nonwoven fabric A in the CD direction is shown in Fig. 9 (a).

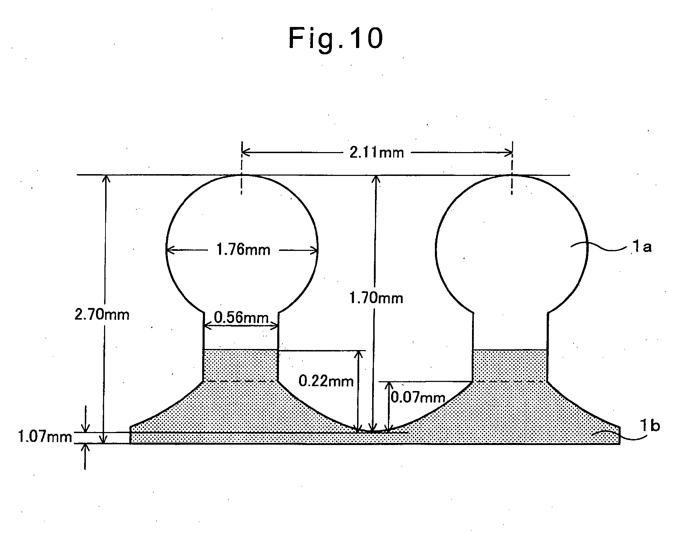

The distance between tips of each two adjacent heights was 2.11 mm. The depths of the recesses formed by each two adjacent heights and the back side section was 1.70 mm. The maximum width of the height tips 21 of the heights in the CD direction was 1.76 mm. The minimum width of the neck sections of the heights in the CD direction was 0.56 mm. The ratio of the maximum width of the height tips of the heights in the CD direction with respect to the minimum width of the neck sections of the heights in the CD direction was 3.14. The thickness of the nonwoven fabric A from the tips of the heights was 2.70 mm. The distance from the border lab between the first nonwoven fabric 1a and the second nonwoven fabric 1b to the border 4 between the heights 2 and the back side section 3 was 9.62% of the distance from the tips 2a of the heights 2 to the border 4 between the heights 2 and the back side section 3. The measurement results for the structure of nonwoven fabric A are shown in summary in Fig. 10.

(2) Comparative Example A

Comparative Example A is a nonwoven fabric having heights formed only in the first nonwoven fabric 1a, as a nonwoven fabric with heights formed according to the prior art. The cross-sectional shapes of the heights are roughly semicircular. A cross-section of Comparative Example A in the CD direction is shown in Fig. 9(b).

The distance between tips of each two adjacent heights was 3.40 mm. The depths of the recesses formed by each two adjacent heights and the base was 0.80 mm. The thickness of Comparative Example A from the tips of the heights was 1.60 mm.

(3) Comparative Example B

Comparative Example B is a nonwoven fabric with a flat front side. A cross-section of Comparative Example B in the CD direction is shown in Fig. 9(c). The thickness of Comparative Example B was 1.00 mm.

A rewet-back test was conducted in the following manner.

(i) A sample was cut to a size of 350 mm × 150 mm.

(ii) The absorbent body used was a commercially available neonatal diaper (Moony Umaretate Shitate by Unicharm Corp.) with the front sheet removed, and the cut sample was placed attached over it. For nonwoven fabric A and Comparative Example A, the height-formed side was oriented upward.

(iii) A 80 milliliter portion of artificial urine (a water-soluble solution of 200 g of urea, 80 g of sodium chloride, 80 g of magnesium sulfate, 8 g of calcium chloride and approximately 1 g of dye Blue #1 in 10 liters of ion-exchanged water) was dropped onto it for 10 seconds.

(iv) It was allowed to stand until the artificial urine permeated and no more artificial urine remained on the front side of the sample.

This was conducted 2 times, and after dropping a total of 160 milliliters,

(v) Filter paper (qualitative filter paper No.2 by Toyo Roshi Co., Ltd.) was placed on the artificial urine-permeated sample and a weight was applied for a load of 35 g/cm$^2$.

(vi) At 60 seconds after application of the load, the filter paper was removed and the weight was measured.

(vii) The weights of the filter paper before and after placement on the sample were compared to calculate the weight of the rewet-back artificial urine.

The results for the rewet-back test conducted as described above were as follows. The weight of the rewet-back artificial urine was 10.78 g for nonwoven fabric A. The weight of the rewet-back artificial urine for Comparative Example A was 16.32 g, and the weight of the rewet-back artificial urine for Comparative Example B was 31.34 g. This confirmed that the effect of minimizing rewet-back with nonwoven fabric A, which was a nonwoven fabric according to an embodiment of the invention, was much higher than with the conventional nonwoven fabrics (Comparative Examples A and B).

Recess load test

When a load is applied to the nonwoven fabric 1 by the wearer, the recess spaces of the nonwoven fabric formed by the heights and the back side section sometimes collapse. In such cases, since no spaces exist to retain the body fluid that has re-wetted by the load by the wearer, the body fluid often exudes from the front side of the nonwoven fabric. The recess spaces must therefore be maintained even when a load has been applied to the nonwoven fabric by the wearer. It was therefore examined whether the recess spaces were maintained in the nonwoven fabric A even under load.

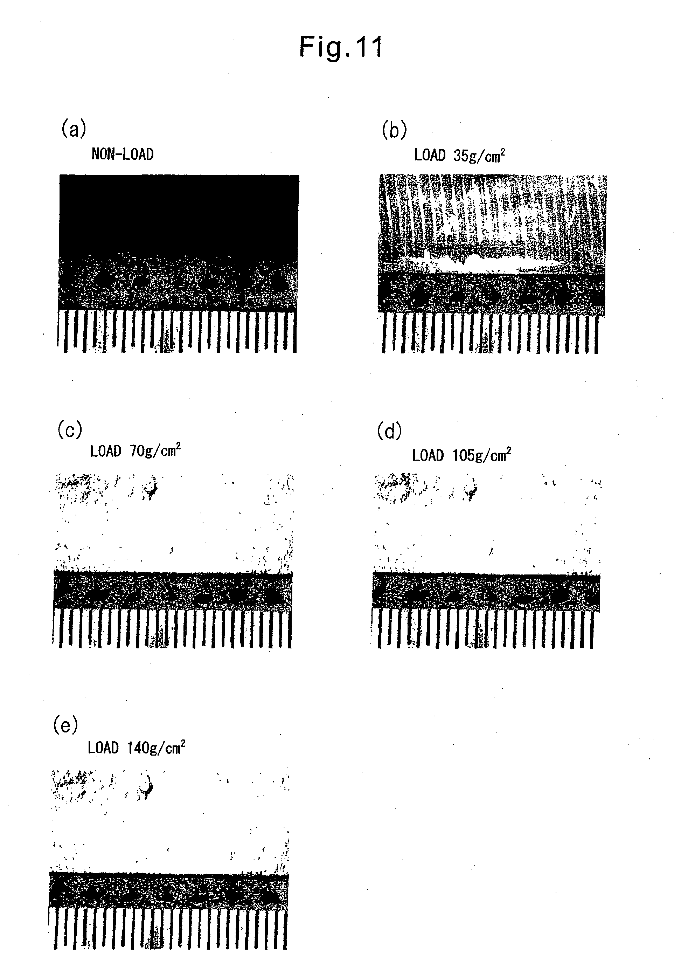

A weight with a flat lower side was placed on the nonwoven fabric A, and a cross-section of the nonwoven fabric in the transverse direction CD was photographed to examine the collapsed condition of the recesses of the nonwoven fabric. Also, the weights were varied to create loads per unit area of 35, 70, 105 and 140 $g/cm^2$, and changes in the collapsed condition of the recesses of the nonwoven fabric by the load per unit area were also examined. The test results are shown in Fig. 11. Fig. 11(a) is a photograph of a cross-section of nonwoven fabric A in the CD direction without placement of the weight, and Figs. 11(b) to (e) are photographs of cross-sections of nonwoven fabric A in the CD direction with application of loads of 35, 70, 105 and 140 $g/cm^2$ on nonwoven fabric A.

As shown in Fig. 11, nonwoven fabric A maintained the recess spaces even with a load per unit area of 140 $g/cm^2$ (see Fig. 11(e)). Also, as shown in Figs. 11(b) to (e), despite some deformation of the height tips of the heights by the load, there was very little deformation of the neck sections.

One example of a very high load by a wearer on an absorbent article is the case where the wearer of a sanitary napkin rides a bicycle. When the wearer rides a bicycle, the pressure applied to the skin of the wearer from the saddle of the bicycle exceeds 100 $g/cm^2$ Since nonwoven fabric A can maintain the recess spaces even under a load per unit area of 140 $g/cm^2$, as shown above, it is predicted that using nonwoven fabric A as the top sheet or second sheet of an absorbent article will not result in rewet-back even when the wearer is riding a bicycle. Furthermore, since the recess spaces are connected in the MD direction of the nonwoven fabric A, air permeability can be ensured by the recess spaces, and it is possible to prevent mustiness between the skin of the wearer and the absorbent article when the wearer is riding a bicycle. Thus, it was confirmed that the recess spaces are maintained in nonwoven fabric A even when a very large load is applied to the absorbent article by the wearer (for example, when riding a bicycle).

Example 2

A nonwoven fabric according to an embodiment of the invention maintains air circulation in the horizontal direction (the direction perpendicular to the thickness direction of the nonwoven fabric A) even under compression with a prescribed pressure. Specifically, a nonwoven fabric sheet of the invention has an air permeation volume of at least 200 $mL/cm^2$ seconds and more preferably 300 to 1000 $mL/cm^2$ seconds in the horizontal direction, under a pressure of 30 $g/cm^2$.

If the air permeation volume in the horizontal direction under a pressure of 30 $g/cm^2$ is at least 200 $mL/cm^2$ seconds, it is possible to effectively prevent mustiness when the absorbent article is worn, and to reliably prevent unpleasantness caused by mustiness, or skin troubles such as itching or rash. In other words, even when the front sheet becomes compressed and comes into close contact with the body of the wearer when the absorbent article is being worn, the fact that circulation of air is sufficiently maintained in the horizontal direction (the direction perpendicular to the thickness direction of the nonwoven fabric) allows the increase in humidity by excretion or sweating during wearing to be reduced, thus constantly providing a comfortable feel during wear without mustiness or rash. In Example 2, the circulation of air in the horizontal direction of a nonwoven fabric according to an embodiment of the invention was confirmed.

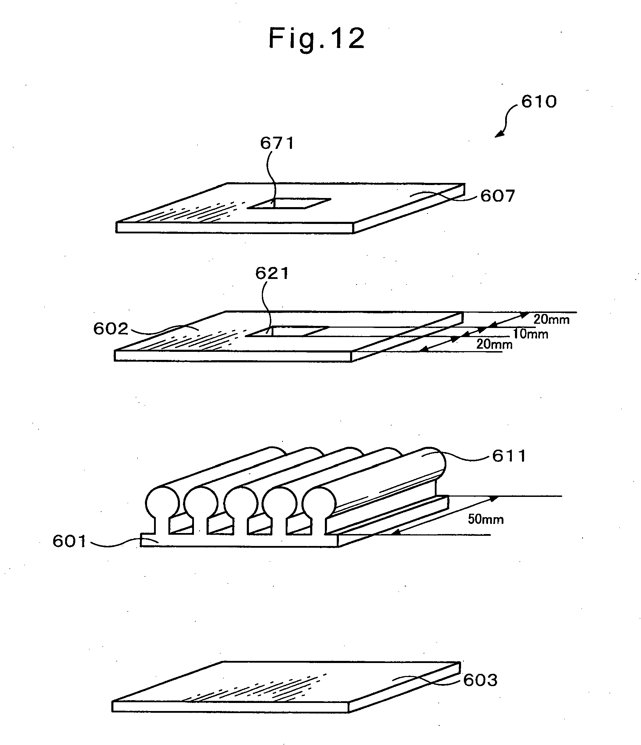

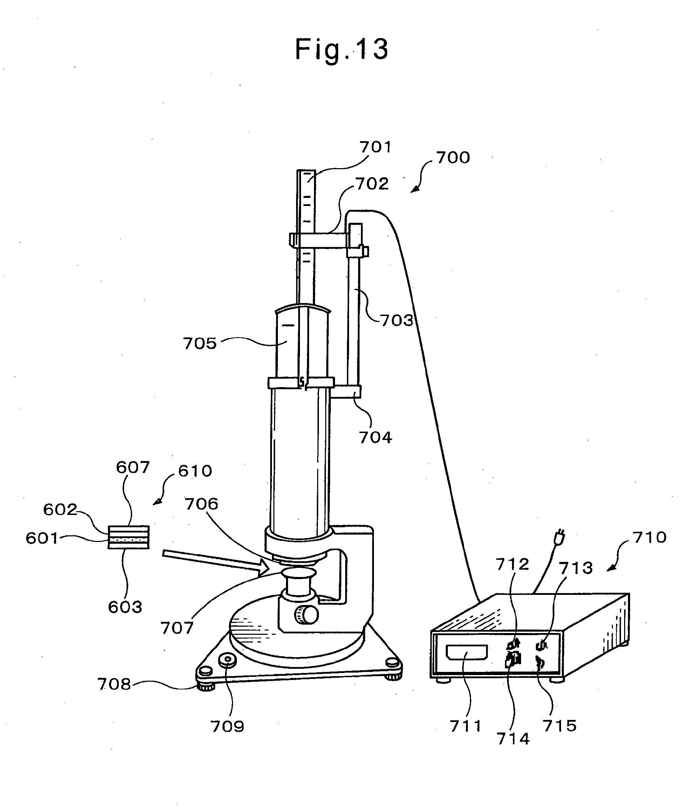

The air permeation volume in the horizontal direction under pressure of 30 $g/cm^2$ on the nonwoven fabric of the embodiment of the invention is measured by the following method. First, the thickness T1 of the nonwoven fabric is measured under a pressure of 30 $g/cm^2$ Also, as shown in Fig. 12, the nonwoven fabric sample 601 is cut into a square with 50 mm sides. The obtained nonwoven fabric sample 601 is inserted between a first acrylic board 602 and a second acrylic board 603, with the side of the nonwoven fabric sample 601 that is to face the skin of the wearer facing the first acrylic board 602 side. The first acrylic board is square, and the dimensions of the first acrylic board are 50 mm × 50 mm x 3 mm, and having at the center an opening 621 that is square with 10 mm sides. The second acrylic board 603 has the same construction as the first acrylic board 602, except for lacking an opening. When air circulation is to be measured, a silicon sheet 607 (hardness: 50) having an opening 671 that is square with 10 mm sides is layered on the upper side of the first acrylic board 602 located between the first acrylic board 602 and the adapter plate 706 (see Fig. 13), to prevent outward leakage of introduced air. The layered silicon sheet 607, first acrylic board 602, nonwoven fabric sample 601 and second acrylic board 603 will hereunder be referred to as the stack 610.

As shown in Fig. 13, the stack 610 is set under the adapter plate 706 of a PA-301 Gurley densometer Model B 700 by Tester Sangyo Co., Ltd., with the silicon sheet 607 side facing upward. The Gurley densometer model B 700 has a volume plate 701, a microphotosensor 702, a microphotosensor strut 703, a microphotosensor strut bracket 704, an inner cylinder 705, an adapter plate 706, a clamping plate 707, a level support 708 and a level tube 709. The clamping plate 707 is raised until the nonwoven fabric sample 601 reaches the aforementioned thickness T1, for adjustment of the clearance. Next, air is introduced at the center section of the nonwoven fabric sample 601 held at thickness T1, through the opening 621, and the time required for introduction of 300 mL of air is measured using a PA-302 digital autocounter 710 by Tester Sangyo Co., Ltd. The digital autocounter 710 comprises a counter 711, a reset switch 712, a pilot lamp 713, a volume switch 714 and a power switch 715. The volume (mL) of air introduced per unit area (1 cm$^2$) $\times$ 1 second through the opening 621 is calculated and recorded as the air permeation volume in the horizontal direction under pressure with a load of 30 g/cm$^2$.

The thickness T1 of the nonwoven fabric under a pressure of 30 g/cm$^2$ can be measured in the following manner. The thickness meter (not shown) used to measure the thickness T1 is a thickness meter having a structure in which two circular horizontal plates are mounted on a stand, allowing measurement to a thickness of 0.01 mm. The upper circular horizontal plate can be moved vertically with respect to the horizontal plane and has a diameter of 44 mm, while the lower circular horizontal plate has a diameter of 100 mm and a smooth surface. The nonwoven fabric is sampled in 10 randomly selected sections at dimensions of 50 mm $\times$ 50 mm (or optionally 45 mm x 45 mm or greater when such dimensions cannot be sampled). A load of the upper circular horizontal plate of the thickness meter is adjusted for a pressure of 30 g/cm$^2$, and the zero position is set. The thickness meter is used to measure the thickness up to 0.01 mm for 10 seconds with a pressure of 30 g/cm$^2$ on the nonwoven fabric. The same measurement is carried out for the rest of the nonwoven fabric, and the average value is calculated to determine the thickness T1 of the sheet under a pressure of 30 g/cm$^2$.

The air permeation volume in the horizontal direction under a pressure of 30 g/cm$^2$ for nonwoven fabric A, as a nonwoven fabric according to an embodiment of the invention, was 391 mL/cm$^2$ seconds, which was a high air permeation volume. For comparison, the air permeation volume was also measured for Comparative Example A and Comparative Example B. The air permeation volume in the horizontal direction under a pressure of 30 g/cm$^2$ for Comparative Example A was 86 mL/cm$^2$ seconds, and the air permeation volume in the horizontal direction under a pressure of 30 g/cm$^2$ for nonwoven fabric B was 8 mL/cm$^2$ seconds. This demonstrated that the air permeation volume in the horizontal direction for nonwoven fabric A was much higher than for Comparative Examples A and B. This indicates that even when pressure is applied by the wearer to nonwoven fabric A as an embodiment of the invention, resulting in close contact, the air permeation volume is high in the horizontal direction, and it is possible to prevent skin problems such as a rash.

Nonwoven fabric 1 of the embodiment described above exhibits the following functions and effects.

(1) It is a nonwoven fabric having a mutually perpendicular MD direction, CD direction and thickness direction, and having in the thickness direction a front side and a back side on the side opposite it, having on the front side heights 2 and recesses 13a formed so as to extend in an alternating fashion parallel to each other in the MD direction, forming repeating corrugations in the CD direction, and on the back side a back side section 3 formed across the entire side, the heights 2 being composed of height tips 21 located at the tips 2a of the heights 2 and neck sections 22 connecting the height tips 21 with the back side section 3, the cross-sectional widths of the neck sections 22 being narrower than the height tips 21 in a cross-section of the heights 2 in the CD direction, and having spaces between the recesses 13a formed by the two neck sections 22 supporting each of the adjacent height tips 21, and the back side section 3. It is thereby possible to minimize "rewet-back" in which the fluid absorbed into the absorbent body exudes out to the front side, even when a load is applied to the nonwoven fabric 1 by the wearer.

(2) In a cross-section in the CD direction of the heights 2, approximate $\Omega$-shape are formed wherein the cross-sectional widths of the neck sections 22 are narrower than the height tips 21, the height tips 21 being the roughly circular sections of the approximate $\Omega$-shapes. This facilitates collapse of the height tips 21 across the CD direction under stress in the thickness direction.

(3) When the height tips 21 collapse across the CD direction by an arbitrary load applied in the thickness direction, adjacent height tips 21 come into close contact with each other so that the spaces between the recesses 13a can be blocked by the height tips 21, neck sections 22 and back side section 3. This allows rewet-back to be further inhibited.

(4) Most of the fibers on the back side section side of the neck sections 22 may be oriented in the thickness direction. This helps prevent collapse of the neck sections 22 under stress in the thickness direction of the nonwoven fabric.

(5) The degree of fiber aggregation in the neck sections 22 is higher than the degree of fiber aggregation in the height tips 21. This allows the height tips 21 to collapse before the neck sections 22, and can produce a structure that prevents the recess spaces formed by each two adjacent neck sections 22 and the back side section 3, from collapsing by the collapsed height tips 21, even under load.

(6) The height tips 21 have a first fiber layer 1a composed of first fibers, the back side section 3 has a second fiber layer 1b composed of second fibers, exhibiting crimping by heat treatment at a temperature at which the first fibers do not exhibit crimping and fusion at the intersections of the first fibers does not occur, and the neck sections 22 have a first fiber layer 1a except on a portion of the back side section 3 side, and a second fiber layer 1b on the back side section 3 side. This allows heights 2 with approximate $\Omega$-shaped cross-sections to be easily formed in the nonwoven fabric 1.

(7) The method for producing the nonwoven fabric comprises a web-forming step in which a second web 12 is formed composed of second fibers that develop crimping by heat treatment, a web-layering step in which a first web 11 is formed composed of first fibers that do not develop crimping at the temperature at which the second fibers develop crimping and that do not undergo fusion at the fiber intersections, and the first web 11 is layered on the second web 12, a recess-forming step in which fibers composing the first web 11 and the fibers composing the second web 12 are divided in the CD direction by a fluid stream, and a plurality of recesses 13a are formed extending parallel to a prescribed direction to a depth reaching from the first web 11 into the second web 12, a heating step in which crimping is produced in the second fibers of the second web 12 by heating, and a fusing step in which the intersections between the first fibers are fused, wherein in the recess-forming step there are created sections where the degree of fiber aggregation of the second fibers is increased directly under the heights 13b formed by the recesses 13a, and in the heating step there are created neck sections in the heights 13b by developing latent crimping by heating at the sections with an increased degree of fiber aggregation of the second fibers directly under the heights 13b formed by the recesses 13a. This allows heights 2 with approximate $\Omega$-shape cross-sections to be easily formed in the nonwoven fabric 1.

(8) In the heating step, the stack 13 of the first web 11 and the second web 12 layered in the web-layering step is stretched to a prescribed stretch factor, the shrinking effect of the second fibers taking place selectively in the CD direction so that the second fibers become bundled directly under the heights 13b. This increases shrinkage of the second web 12 in the CD direction, and allows heights 2 with approximate $\Omega$-shaped cross-sections to be reliably formed in the nonwoven fabric 1.

(9) In the recess-forming step, the blowing direction of the nozzles of the blower 302 that produces the fluid stream 303 is pointed in the direction opposite the traveling direction of the stack 13 of the first web 11 and the second web 12, to orient the constituent fibers of the stack 13 in the CD direction. This increases shrinkage of the second web 12 in the CD direction, and allows heights 2 with approximate $\Omega$-shaped cross-sections to be reliably formed in the nonwoven fabric 1.

(10) Even when stress is applied to the height tips 21 from the thickness direction, the adjacent height tips 21 that have deformed in the CD direction do not only contact each other, but also mutually produce force in the CD direction, so that the height tips 21 do not completely collapse in the thickness direction. Consequently, the height tips 21 can act as shock-absorbers so that the wearer does not feel the hardness of the neck sections 22.

The nonwoven fabric 1 of the embodiment described above may further be modified as follows.

(1) So long as the height tips 21 of the heights 2 collapse across the transverse direction, and the adjacent deformed height tips 21 support each other while maintaining recess spaces formed by the two adjacent neck sections and the back side section, the cross-sectional shapes of the heights 2 are not limited to approximate $\Omega$-shapes. For example, when the height tips 21 are constructed so as to collapse in the transverse direction and significantly spread out, the cross-sectional shapes of the heights 2 may be rectangular shapes. Also, the cross-sectional shapes of the heights 2 may be mushroom-shaped, with the height tips 21 formed in an umbrella fashion.

(2) If it is possible to minimize "rewet-back" in which the fluid absorbed into the absorbent body exudes out to the front side even when a load is applied to the nonwoven fabric 1 by the wearer, the height tips 21 of some of the heights 2 may collapse across the transverse direction, or all of them may collapse across that direction.

Any of the aforementioned embodiments may also be applied in combination with the modifications. The modifications may also be applied in combination with each other.

The explanation above is merely intended as an example, and the invention is in no way restricted by the described embodiment.

Explanation of Symbols

| 1 | Nonwoven fabric |
|---|---|
| 1a | First fiber layer |
| 1b | Second fiber layer |
| 1ab | Interface between first fiber layer and second fiber layer |
| 2 | Height |
| 3 | Back side section |
| 4 | Interface between height and back side section |
| 11 | First web |

| 12 | Second web |
|---|---|
| 13 | Stack |
| 13a | Recess |
| 13b | Height |
| 21 | Height tip |
| 22 | Neck section |
| 100, 200 | Roller cards |
| 101, 201 | Feeders |
| 102, 202 | Cylinders |
| 103, 203 | Walkers |
| 104, 204 | Doffers |
| 105, 205 | Condensers |
| 106, 206, 405, 601, 602 | Conveyors |
| 301 | Suction drum |
| 302 | Blower |
| 302a | Nozzle |
| 303 | Fluid stream |
| 400 | Heat setting machine |
| 401-404 | Heat setting chambers |
| 500 | Take-up drum |
| 601 | Nonwoven fabric sample |
| 602 | First acrylic board |
| 603 | Second acrylic board |
| 607 | Silicon sheet |
| 610 | Stack |
| 700 | Gurley densometer |
| 706 | Adapter plate |
| 707 | Clamping plate |
| 710 | Digital autocounter |

**Claims**

1.  A nonwoven fabric having a mutually perpendicular longitudinal direction, transverse direction and thickness direction, and having in the thickness direction a front side and a back side on the side opposite it, having on the front side heights and recesses formed so as to extend in an alternating fashion parallel to each other in the longitudinal direction, forming repeating corrugations in the transverse direction, and on the back side a back side section formed across the entire side,
    the heights being composed of height tips located at the tips of the heights and neck sections connecting the height tips with the back side section,
    the cross-sectional widths of the neck sections being narrower than the height tips in a cross-section of the heights in the transverse direction, and having recess spaces formed by the two neck sections supporting each of the adjacent height tips, and the back side section.

2.  The nonwoven fabric according to claim 1, wherein:

    the cross-sections of the heights in the transverse direction are approximate $\Omega$-shapes in which the cross-sectional widths of the neck sections are narrower than the height tips,
    the height tips being the roughly circular sections of the approximate $\Omega$-shapes.

3.  The nonwoven fabric according to claim 1 or 2, wherein the height tips collapse across the transverse direction by an arbitrary load applied in the thickness direction, such that adjacent height tips come into close contact with each other, and the spaces between the recesses can be blocked by the height tips, neck sections and back side section.

4.  The nonwoven fabric according to any one of claims 1 to 3, wherein most of the fibers on the back side section side of the neck sections are oriented in the thickness direction.

5.  The nonwoven fabric according to any one of claims 1 to 4, wherein the degree of fiber aggregation in the neck sections is higher than the degree of fiber aggregation in the height tips.

6. The nonwoven fabric according to any one of claims 1 to 5, wherein:

the height tips have a first fiber layer composed of first fibers,
the back side section has a second fiber layer composed of second fibers, exhibiting crimping by heat treatment at a temperature at which the first fibers do not exhibit crimping and fusion at the intersections of the first fibers does not occur, and
the neck sections have a first fiber layer except on a portion of the back side section side, and a second fiber layer on the back side section side.

7. An absorbent article wherein a nonwoven fabric according to any one of claims 1 to 6 is used as a top sheet which is to contact with the skin of a wearer, or a second sheet to be situated between a top sheet and an absorbent body.

8. A method for producing a nonwoven fabric, comprising:

a web-forming step in which a second web is formed composed of second fibers that develop crimping by heat treatment,
a web-layering step in which a first web is formed composed of first fibers that do not develop crimping at the temperature at which the second fibers develop crimping and that do not undergo fusion at the fiber intersections, and the first web is layered on the second web,
a recess-forming step in which the first fibers composing the first web and the second fibers composing the second web are divided in the CD direction by a fluid stream, and a plurality of recesses are formed extending parallel to a prescribed direction to a depth reaching from the first web into the second web,
a heating step in which crimping is produced in the second fibers of the second web by heating, and
a fusing step in which the intersections between the first fibers are fused,
wherein in the recess-forming step there are created sections where the degree of fiber aggregation of the second fibers is increased directly under the heights formed by the recesses, and
in the heating step there are created neck sections in the heights by developing latent crimping by heating at the sections that have an increased degree of fiber aggregation of the second fibers directly under the heights formed by the recesses.

9. The method for producing a nonwoven fabric according to claim 8, wherein in the heating step, the stack of the first web and the second web layered in the web-layering step is stretched to a prescribed stretch factor, the shrinking effect of the second fibers taking place selectively in the CD direction so that the second fibers become bundled directly under the heights.

10. The method for producing a nonwoven fabric according to claim 8 or 9, wherein in the recess-forming step, the blowing direction of the nozzles of the blower that produces the fluid stream is pointed in the direction opposite the traveling direction of the stack of the first web and the second web, to orient the constituent fibers of the stack in the CD direction.

**Patentansprüche**

1. Vliesstoff mit einer gemeinsamen senkrechten Längsrichtung, Querrichtung und Dickenrichtung, und in Dickenrichtung eine Vorderseite und eine Rückseite auf der gegenüberliegenden Seite davon haben, die auf der Vorderseite Höhen und Vertiefungen haben, die so geformt sind, dass sie sich auf eine abwechselnde Weise parallel zueinander in Längsrichtung erstrecken und wiederholte Wellen in Querrichtung formen, und auf der Rückseite einen Rückseitenabschnitt quer über die gesamte Seite formen,
wobei die Höhen aus Höhenspitzen zusammengesestzt sind, die an den Spitzen der Höhen und Halsabschnitte platziert sind, die die Höhenspitzen mit dem Rückseitenabschnitt verbunden sind,
die querschnittlichen Breiten des Halsabschnitts schmaler sind als die Höhenspitzen in einem Querschnitt der Höhen in Querrichtung, und Vertiefungsräume haben, die von den zwei Halsabschnitten geformt sind, die jede der angrenzenden Höhenspitzen und die hinteren Seitenabschnitte stützen.

2. Vliesstoff nach Anspruch 1, wobei
die Querschnitte der Höhen in Querrichtung ungefähre Ω-Formen sind, in denen die Querschnittsbreiten des Halsabschnitts schmaler als die Höhenspitzen sind,
wobei die Höhenspitzen ungefähr kreisförmige Abschnitte der etwaigen $\Omega$-Formen sind,

3. Vliesstoff nach Anspruch 1 oder 2, wobei die Höhenspitzen quer über die Querrichtung durch eine willkürliche Last zusammenbrechen, die in Dickenrichtung aufgetragen wurde, sodass die angrenzenden Höhenspitzen in engen Kontakt miteinander kommen, und die Zwischenräume zwischen den Vertiefungen von den Höhenspitzen, Halsabschnitten und den hinteren Seitenabschnitt blockiert werden können.

4. Vliesstoff nach einem der Ansprüche 1 bis 3, wobei die meisten Fasern am Rückseitenabschnitt des Halsabschnitts in Dickenrichtung ausgerichtet sind.

5. Vliesstoff nach einem der Ansprüche 1 bis 4, wobei der Grad der Faseransammlung in den Halsabschnitten höher ist, als der Grad der Faseransammlung in den Höhenspitzen.

6. Vliesstoff nach einem der Ansprüche 1 bis 5, wobei
die Höhenspitzen eine erste Faserschicht haben, die aus ersten Fasern zusammengesetzt sind,
der Hinterseitenabschnitt eine zweite Faserschicht hat, die aus zweiten Fasern zusammengesetzt sind, und ein Crimpen durch Hitzebehandlung darstellen, und zwar bei einer Temperatur, bei der die ersten Fasern kein Crimpen darstellen und keine Verschmelzungen an den Schnittpunkten der ersten Fasern geschehen, und
die Halsabschnitte eine erste Faserschicht haben, mit Ausnahme eines Abschnitts an der Seite des Rückseitenbereichs, und eine zweite Faserschicht an der Seite des Rückseitenabschnitts.

7. Saugfähiger Artikel, worin ein Vliesstoff nach einem der Ansprüche 1 bis 6 als oberste Schicht verwendet wird, die mit der Haut des Trägers in Kontakt ist, oder eine zweite Schicht, die zwischen einer obersten Schicht und einem saugfähigen Körper gelegen ist.

8. Verfahren zur Herstellung eines Vliesstoffs, der Folgendes umfasst:

einen gewebebildenden Schritt, in dem ein zweites Gewebe geformt wird, das aus zweiten Fasern zusammengesetzt ist, die ein Crimpen durch Hitzebehandlung entwickeln,
ein gewebeschichteter Schritt, in dem ein erstes Gewebe geformt wird, das aus ersten Fasern zusammengesetzt ist, die kein Crimpen bei der Temperatur, bei der die zweiten Fasern ein Crimpen entwickeln, und die keiner Schmelzung an den Faserschnittpunkten unterzogen wird, und das erste Gewebe auf dem zweiten Gewebe geschichtet ist,
ein vertiefungsförmiger Schritt, in dem die ersten Fasern das erste Gewebe zusammensetzen, und die zweiten Fasern das zweite Gewebe zusammensetzen, in der zweiten CD-Richtung durch einen Flüssigkeitsstrom geteilt werden, und die Vielzahl von Vertiefungen geformt werden, die sich parallel zur beschriebenen Richtung erstrecken, und zwar auf eine Tiefe, die vom ersten Gewebe in das zweite Gewebe reichen,
ein Erhitzungsschritt, in dem Crimpen in den zweiten Fasern des zweiten Gewebes durch Erhitzen produziert wird, und
ein Verschmelzungsschritt, in dem die Schnittpunkte zwischen den ersten Fasern verschmolzen werden,
wobei im Vertiefungen bildenen Schritt Abschnitte geschaffen werden, wobei der Grad an Faseransammlung der zweiten Fasern zunehmend direkt unter den Höhen, die von dem Vertiefungen geformt werden, erhöht wird, und
im Erhitzungsschritt geschaffene Halsabschnitte in den Höhen durch Entwickeln eines ruhenden Crimpens durch Erhitzen an den Abschnitten, die einen erhöhten Grad an Faseransammlung der zweiten Fasern direkt unter der Höhe haben, die von den Vertiefungen geformt wurden.

9. Verfahren zur Herstellung eines Vliesstoffs nach Anspruch 8, wobei beim Erhitzungsschritt der Stapel des ersten Gewebes und des zweiten Gewebes, die im Gewebe geschichteten Schritt geschichtet sind, bis zu einem vorgeschriebenen Dehnungsfaktor gedehnt wird, wobei die Schrumpfauswirkung der zweiten Fasern selektiv in CD-Richtung stattfinden, sodass die zweiten Fasern direkt unter den Höhen gebündelt werden.

10. Verfahren zum Herstellen eines Vliesstoffs nach Anspruch 8 oder 9, wobei in dem die Vertiefung bildenen Schritt, die Blasrichtung der Düsen des Gebläses, das einen Flüssigkeitsstrom produziert, in die Richtung gewiesen wird, die gegenüber der Bewegungsrichtung des Stapels des ersten Gewebes und des zweiten Gewebes ist, um den Bestandteil der Fasern des Stapels in CD-Richtung auszurichten.

**Revendications**

1. Tissu non tissé ayant une direction allant dans le sens longitudinal, une direction allant dans le sens transversal et une direction allant dans le sens de l'épaisseur mutuellement perpendiculaires, et ayant dans la direction allant dans le sens de l'épaisseur une face avant et une face arrière du côté opposé à celle-ci, ayant sur la face avant des parties hautes et des évidements formés de manière à s'étendre parallèlement de façon alternée les uns par rapport aux autres dans la direction allant dans le sens longitudinal, formant des ondulations se répétant dans la direction allant dans le sens transversal, et sur la face arrière une section de face arrière formée en travers de toute la face,

   les parties hautes étant composées de pointes de partie haute situées au niveau des pointes des parties hautes et des sections de col raccordant les pointes de partie haute au niveau de la section de face arrière,

   les largeurs en coupe des sections de col étant plus étroites par rapport aux pointes de partie haute dans une coupe transversale des parties hautes dans la direction allant dans le sens transversal, et ayant des espaces d'évidement formés par les deux sections de col supportant chacune des pointes de partie haute adjacentes, et la section de face arrière.

2. Tissu non tissé selon la revendication 1, dans lequel :

   les sections en coupe des parties hautes dans la direction allant dans le sens transversal sont des formes approximativement en Ω où les largeurs en coupe des sections de col sont plus étroites par rapport aux pointes de partie haute,

   les pointes de partie haute étant les sections plus ou moins circulaires des formes approximativement en Ω.

3. Tissu non tissé selon la revendication 1 ou la revendication 2, dans lequel les pointes de partie haute s'affaissent dans la direction allant dans le sens transversal sous l'effet d'une charge arbitraire appliquée dans la direction allant dans le sens de l'épaisseur, de telle sorte que des pointes de partie haute adjacentes entrent en contact étroit les unes par rapport aux autres, et les espaces entre les évidements peuvent être bloqués par les pointes de partie haute, les sections de col et la section de face arrière.

4. Tissu non tissé selon l'une quelconque des revendications 1 à 3, dans lequel la plupart des fibres au niveau de la section de face arrière des sections de col sont orientées dans la direction allant dans le sens de l'épaisseur.

5. Tissu non tissé selon l'une quelconque des revendications 1 à 4, dans lequel le degré d'agrégation des fibres dans les sections de col est supérieur au degré d'agrégation des fibres dans les pointes de partie haute.

6. Tissu non tissé selon l'une quelconque des revendications 1 à 5, dans lequel :

   les pointes de partie haute ont une première couche de fibres composée de premières fibres,

   la section de face arrière a une deuxième couche de fibres composée de deuxièmes fibres, présentant une frisure par traitement thermique à une température à laquelle les premières fibres ne présentent pas de frisure et le soudage au niveau des intersections des premières fibres ne se produit pas, et

   les sections de col ont une première couche de fibres sauf sur une partie du côté de la section de face arrière, et une deuxième couche de fibres du côté de la section de face arrière.

7. Article absorbant dans lequel un tissu non tissé selon l'une quelconque des revendications 1 à 6 est utilisé comme feuille de dessus qui est en contact avec la peau d'un utilisateur, ou deuxième feuille devant être située entre une feuille de dessus et un corps absorbant.

8. Procédé de fabrication d'un tissu non tissé, comportant :

   une étape consistant à former un voile au cours de laquelle un deuxième voile est formé composé de deuxièmes fibres qui développent une frisure par traitement thermique,

   une étape consistant à réaliser des couches de voile au cours de laquelle un premier voile est formé composé de premières fibres qui ne développent pas de frisure à la température à laquelle les deuxièmes fibres développent une frisure et qui ne subissent pas de soudage au niveau des intersections de fibres, et le premier voile est placé en couche sur le deuxième voile,

   une étape consistant à former des évidements au cours de laquelle les premières fibres composant le premier voile et les deuxièmes fibres composant le deuxième voile sont divisées dans la direction allant dans le sens transversal par un flux fluide, et une pluralité d'évidements sont formés s'étendant parallèlement par rapport à une direction prescrite relativement à une profondeur allant du premier voile jusque dans le deuxième voile,

une étape consistant à chauffer au cours de laquelle la frisure se produit dans les deuxièmes fibres du deuxième voile par chauffage, et

une étape consistant à souder au cours de laquelle les intersections entre les premières fibres sont soudées, dans lequel au cours de l'étape consistant à former des évidements il y a des sections qui sont créées où le degré d'agrégation des fibres des deuxièmes fibres est augmenté directement sous les parties hautes formées par les évidements, et

au cours de l'étape consistant à chauffer, il y a des sections de col qui sont créées dans les parties hautes par le développement d'une frisure latente par le chauffage au niveau des sections qui ont un degré d'agrégation des fibres supérieur des deuxièmes fibres directement sous les parties hautes formées par les évidements.

9. Procédé de fabrication d'un tissu non tissé selon la revendication 8, dans lequel, au cours de l'étape consistant à chauffer, la pile du premier voile et du deuxième voile mis en couches au cours de l'étape consistant à réaliser des couches de voile est étendue selon un facteur d'extension prescrit, l'effet de rétrécissement des deuxièmes fibres ayant lieu de manière sélective dans la direction allant dans le sens transversal de telle sorte que les deuxièmes fibres finissent par se mettre en faisceaux directement sous les parties hautes.

10. Procédé de fabrication d'un tissu non tissé selon la revendication 8 ou la revendication 9, dans lequel au cours de l'étape consistant à former des évidements, la direction de soufflage des buses du souffleur qui produit le flux fluide est dirigée dans la direction allant à l'opposé de la direction d'avance de la pile du premier voile et du deuxième voile, pour orienter les fibres constituantes de la pile dans la direction allant dans le sens transversal.

# Fig.1

(a)

(b)

EP 2 612 960 B1

# Fig.2

STEP A

STEP B

STEP C

STEP D

STEP E

# Fig.3

(a)

(b)

(c)

(d)

## Fig.4

## Fig.5

# Fig.6

305

$\theta$

302

303

304

13

MOVEMENT DIRECTION

301a

301

# Fig.7

(a)

(b)

LOAD P

# Fig.8

# Fig.9

NONWOVEN FABRIC

(a)

COMPARATIVE EXAMPLE A

(b)

COMPARATIVE EXAMPLE B

(c)

# Fig.10

# Fig.11

(a)
NON-LOAD

(b)
LOAD 35g/cm$^2$

(c)
LOAD 70g/cm$^2$

(d)
LOAD 105g/cm$^2$

(e)
LOAD 140g/cm$^2$

# Fig.12

610

671

607

621

602

20mm
10mm
20mm

611

601

50mm

603

# Fig.13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008025082 A